# EUROPEAN PATENT APPLICATION

(11) **EP 2 860 593 A1**
(43) Date of publication of application: **15.04.2015**
(21) Application number: 14188086.4
(22) Date of filing: 08.10.2014
(51) Int. Cl.: G04B 37/14, G04B 37/18, G04B 37/00, A44C 5/14, A61B 5/00, G04B 45/00

(54) **Biological information measurement device**

(30) Priority: 11.10.2013 JP 2013213478
(71) Applicant: Seiko Epson Corporation, Shinjuku-ku, Tokyo 163-0811 (JP)
(72) Inventor: Shibuya, Junya, Nagano, 392-8502 (JP); Hidai, Yoshihiro, Nagano, 392-8502 (JP); Tanaka, Shigemitsu, Nagano, 392-8502 (JP)
(74) Representative: Hoffmann Eitle

(57) **Abstract**

A biological information measurement device includes: a device main body; and a band mounted on the device main body by a connection portion, wherein the device main body and the band are connected with a miter joint, oblique sides are provided at end portions of the device main body to which the band is connected and the connection portion, and the band is connected to the device main body with the oblique side on the device main body side and the oblique side on the connection portion side facing each other.

## Description

### BACKGROUND

### 1. Technical Field

The present invention relates to a biological information measurement device which is mounted on a test object so as to measure biological information.

### 2. Related Art

In the past, an electronic device such as a measurement device which is mounted on the wrist or the like of a wearer and measures biological information such as the pulse or a wristwatch having a function of measuring the biological information has been known. In such an electronic device, it is necessary to bring a device main body which performs measurement into close contact with an appropriate site such as a wrist of the wearer. A band which is mounted on a main body of such a measurement device or electronic device and provided with a stretchable belt portion is disclosed in JP-A-2012-90975.

The band described in JP-A-2012-90975 is provided with first and second band members which are mounted on a device main body, and a connecting member. Each band member has a stretchable portion which expands and contracts along a band extension direction, and the stretchable portion has flexibility and has a first slit having a pair of slits following a width direction and a slit following the band extension direction, and a pair of second slits communicating with the outside of the stretchable portion along the width direction. The connecting member is provided with a fixed member, a sliding member slidable along the band extension direction of the first band member, and a biasing member which biases the sliding member in the opposite direction to the band extension direction, and the sliding member is provided with a connection portion (a projecting bar) which is connected to the second band member.

When mounting such a biological information measurement device on the wrist of a test object, a tensile force acts on the band by an operation of a wearer, and a device main body is mounted on the wrist in a state where the stretchable portion is extended by the tensile force. Then, if the wearer removes the band from the hand, a restoring force acts on the stretchable portion. In this way, the band is tightened up, and thus it is possible to bring the device main body into close contact with the wrist regardless of the degree of tightening of the device main body to the wrist by the wearer.

However, the band provided at the biological information measurement device described in JP-A-2012-90975 described above is thin compared to the width of the device main body in a case of being viewed in plan from a vertical direction with respect to a display surface of a display section provided at the biological information measurement device and a device main body section is visible relatively greatly, and therefore, there is a problem in that a visual feeling of pressure is given to the wearer. Therefore, there is also a concern that due to a feeling of pressure from a visual sensation, it may be difficult to continue to measure the biological information of a measurement object over a long period of time. Further, the width of the device main body is wider than the belt portion and the device main body is large, and therefore, an inertial force acting when a test obj ect moves increases, and therefore, there is also a concern of adversely affecting the measurement of biological information. In addition, the display surface is increased, whereby the visual recognition of the measured biological information is easy. However, power consumption of a display device configuring the display surface increases, and thus there is also a problem in that prolonged measurement is hindered.

### SUMMARY

An advantage of some aspects of the invention is to solve at least a part of the problems described above, and the invention can be implemented as the following forms or application examples.

### Application Example 1

This application example of the invention is directed to a biological information measurement device including: a device main body; and a band connected to the device main body, wherein oblique sides are provided at end portions of the device main body to which the band is connected and the band, and the band is connected to the device main body with the oblique side of the device main body and the oblique side of the band facing each other.

According to such a biological information measurement device, the oblique sides are provided at the end portions of the device main body and the band, and by making the oblique sides face each other, it is possible to connect the device main body and the band by a miter joint. Since the device main body and the band are connected by a miter joint, it is possible to cause the widths of the device main body and the band to continuously change. Therefore, it is possible to secure a visual feeling of unity of the device main body and the band and it is possible to make the device main body be viewed as small, and therefore, it is possible to suppress (alleviate) a visual feeling of pressure due to prolonged wearing. Further, the shape of the biological information measurement device changes continuously and smoothly, whereby, for example, even when a test obj ect is in motion with the device main body mounted on an arm or the like, it is possible to suppress the device main body from being caught in a sleeve of clothing. Therefore, the influence on the biological information of a person with the biological information measurement device mounted thereon is suppressed, and thus it is possible to obtain biological information with high precision over a long period of time.

### Application Example 2

It is preferable that the biological information measurement device according to the application example described abovwe further includes a connection portion which connects the device main body and the band.

According to such a biological information measurement device, the device main body and the band are connected through the connection portion, whereby the connection can be made solidly compared to a case of directly connecting the band to the device main body. Therefore, it is possible to suppress the band from being detached from the device main body due to the motion of a test object.

### Application Example 3

In the biological information measurement device according to the application example described above, it is preferable that a bottomed groove portion is provided between the oblique side of the device main body and the oblique side of the band.

According to such a biological information measurement device, the groove portion is provided between the device main body and the connection portion, whereby it is possible to suppress wear due to the contact between the device main body and the connection portion when the band expands and contracts according to the motion of a person with the biological information measurement device mounted thereon. Further, the groove portion is provided, whereby it is possible to set a permissible amount of variation in intersection at the time of the manufacturing of the device main body or the band to be wider compared to a case where there is no groove portion, and therefore, it is possible to improve yield at the time of the manufacturing.

### Application Example 4

In the biological information measurement device according to the application example described above, it is preferable that the device main body is provided with a case in which a module with an electronic circuit installed therein is accommodated and has a front and a back, an area with a film which includes metal provided therein is provided on a front surface of the case, and the film and the module are electrically connected.

According to such a biological information measurement device, the film which includes metal is provided on the front surface of the case and the film and the module are connected, whereby it is possible to use the film as an antenna of a communication section configured by an electronic circuit. The film is provided on the front surface of the case, whereby it is possible to perform stable wireless communication compared to a case where an antenna is provided inside the case. Further, the film is provided on the front surface of the case, whereby it is possible to increase the strength of the case.

### Application Example 5

In the biological information measurement device according to the application example described above, it is preferable that the module is provided with a first display portion fitted into a window portion having a quadrangular shape provided with an upper base and a lower base extending in a second direction intersecting a first direction in which the band connected to the device main body extends, on the front surface of the case, and the first display portion is covered with a cover body having a parallelogram shape provided with an upper base and a lower base becoming a set of opposite sides extending in the second direction.

In such a biological information measurement device, the first display portion fitted into the window portion having a quadrangular shape is covered with the cover body having a parallelogram shape, whereby each side of the first display portion and the window portion can be surrounded by each side of the cover body. The first display portion and the window portion are surrounded by each side of the cover body having a parallelogram shape, whereby it is possible to make the first display portion and the window portion be viewed as large when viewing the device main body, and thus it is possible to suppress a feeling of pressure which is given to a person with the biological information measurement device mounted thereon. Further, it is possible to make the first display portion which is small compared to the device main body be viewed as large. Therefore, a test object can mount the biological information measurement device without feeling great stress, and thus it is possible to measure biological information over a longer period of time. By continuing to measure the biological information of a test object over a long period of time, it becomes easy for the test object to more accurately grasp one's own state.

### Application Example 6

In the biological information measurement device according to the application example described above, it is preferable that the opposite sides of the cover body are disposed side by side with the oblique sides on the connection portion side and the opposite sides are in contact with the upper base and the lower base of the window portion at an acute angle.

According to such a biological information measurement device, the upper base and the lower base of the window portion and the opposite sides of the cover body are in contact with each other at an acute angle, and therefore, areas having internal angles in which the upper base and the opposite side are in contact with each other and the lower base and the opposite side are in contact with each other are formed. Due to these areas, it is possible to make the first display portion and the window portion be viewed as large when viewing the device main body, and thus it is possible to suppress a feeling of pressure which is given to a person with the biological information measurement device mounted thereon.

Therefore, a test object can mount the biological information measurement device without feeling great stress, and thus it is possible to measure biological information over a longer period of time. By continuing to measure the biological information of a test object over a long period of time, it becomes easy for the test object to more accurately grasp one's own state.

### Application Example 7

In the biological information measurement device according to the application example described above, it is preferable that the module is provided with a second display portion fitted into a hole portion provided in the front surface of the case and the second display portion is covered with the cover body.

According to such a biological information measurement device, it is possible to display a variety of information obtained in the biological information measurement device or an operation state on the first display portion and the second display portion in different display ways. Further, it is possible to simultaneously display different information related to each other. Therefore, since it is possible to provide necessary information even by a small biological information measurement device, it is possible to improve convenience.

### Application Example 8

In the biological information measurement device according to the application example described above, it is preferable that the second display portion is provided at an area on an internal angle side in which the lower base of the window portion and the opposite side are in contact with each other at an acute angle.

According to such a biological information measurement device, the second display portion is provided at an area on an internal angle side in which the lower base of the window portion and the opposite side of the cover body are in contact with each other at an acute angle. Therefore, it is possible to display a variety of information obtained in the biological information measurement device or an operation state by using a plurality of display ways and to suppress the surface area of the device main body.

### Application Example 9

In the biological information measurement device according to the application example described above, it is preferable that the module has a control section, an operation section providing a command is connected to the control section, the operation section is provided at least one in the three o'clock-side side surface of the case section in a case where the window portion is viewed in plan from a vertical direction and on an extension line of the opposite side.

According to such a biological information measurement device, the operation section connected to the control section is provided in the three o'clock-side side surface in a case where the case section is viewed in plan, on an extension line of the opposite side of the cover body. Therefore, when a person with the biological information measurement device mounted thereon operates the device, the person can intuitively touch the operation section by visually recognizing the opposite side of the cover body, and thus it is possible to improve operability.

### Application Example 10

In the biological information measurement device according to the application example described above, it is preferable that the module is provided with a power supply section having a battery which drives the control section or the first display portion and the second display portion, and a terminal for communication to output data stored in the control section to the outside and a terminal for charging to charge the battery are connected to the module section, and the terminals are disposed in the nine o'clock-side side surface of the case section in a case where the window portion is viewed in plan from a vertical direction.

In such a biological information measurement device, the terminal for charging and the terminal for communication are disposed in the nine o'clock-side side surface of the case section in a case where the case section is viewed in plan. Therefore, when operating the operation section provided in the three o'clock-side side surface, it is possible to suppress touch on these terminals. Therefore, it is possible to prevent contamination of the terminal for charging and the terminal for communication, and thus it is possible to improve the reliability of charging and communication.

### Application Example 11

In the biological information measurement device according to the application example described above, it is preferable that the terminals are configured to include two or more terminals for charging and two or more terminals for communication, and a distance between the terminals for charging and a distance between the terminals for communication are different from each other.

In such a biological information measurement device, each of the terminal for charging and the terminal for communication is configured with two or more terminals, and a disposition interval of the terminals for charging and a disposition interval of the terminals for communication are different from each other. Therefore, it is possible to prevent a short circuit by widening the disposition interval of the terminals for charging and to increase disposition density by narrowing the disposition interval of the terminals for communication. Therefore, it is possible to improve the reliability of the biological information measurement device and attain a reduction in the size of the biological information measurement device.

### Application Example 12

In the biological information measurement device according to the application example described above, it is preferable that in the terminals, the terminal for charging is disposed outside the terminal for communication.

According to such a biological information measurement device, the terminal for charging is disposed outside the terminal for communication, and therefore, it is possible to further suppress a short circuit between the terminals for charging. Therefore, it is possible to further improve the reliability of the biological information measurement device.

### Application Example 13

In the biological information measurement device according to the application example described above, it is preferable that a material which includes stainless steel is used for the terminals.

According to such a biological information measurement device, a material which includes stainless steel is used for the terminals. Therefore, it is possible to prevent the terminals from corroding due to sweat or the like of a test object with the biological information measurement device mounted thereon. Further, it is possible to easily perform cleaning when the terminals are dirty.

### Application Example 14

In the biological information measurement device according to the application example described above, it is preferable that tips of the terminals are exposed on a side surface of the case or protrude from the side surface of the case.

According to such a biological information measurement device, the terminals are exposed on or protrude from the side surface of the case, whereby it is possible to prevent dirt from sticking to the terminals and easily clean off dirt.

### Application Example 15

In the biological information measurement device according to the application example described above, it is preferable that the case section is provided with a positioning hole which is used when the terminal is connected to an external device, and the positioning hole is disposed on the same line as the terminals.

According to such a biological information measurement device, the positioning hole which is used when being connected to an external device is disposed on the same line as the terminals provided in the case section. Therefore, by using the positioning hole, it is possible to easily connect the biological information measurement device and the external device.

### BRIEF DESCRIPTION OF THE DRAWINGS

The invention will be described with reference to the accompanying drawings, wherein like numbers reference like elements.
Fig. 1 is a top view schematically showing the external appearance of a biological information measurement device according to an embodiment.
Figs. 2A and 2B are perspective views schematically showing the external appearance of the biological information measurement device according to the embodiment.
Fig. 3 is a development view schematically showing the structure of the biological information measurement device according to the embodiment.
Fig. 4 is an enlarged view schematically showing the structure of a main body section of the biological information measurement device.
Figs. 5A and 5B are side views schematically showing the nine o'clock-side side surface of the main body section of the biological information measurement device.
Figs. 6A and 6B are a plan view and a cross-sectional view schematically showing a first band portion.
Figs. 7A to 7C are a plan view and a cross-sectional view schematically showing a second band portion.

### DESCRIPTION OF EXEMPLARY EMBODIMENTS

Hereinafter, an embodiment of the invention will be described using the drawings. In addition, in each drawing shown below, in order to show each constituent element in size to the extent that can be recognized on the drawing, there is a case where the dimension or the ratio of each constituent element is described differently from that of the actual constituent element.

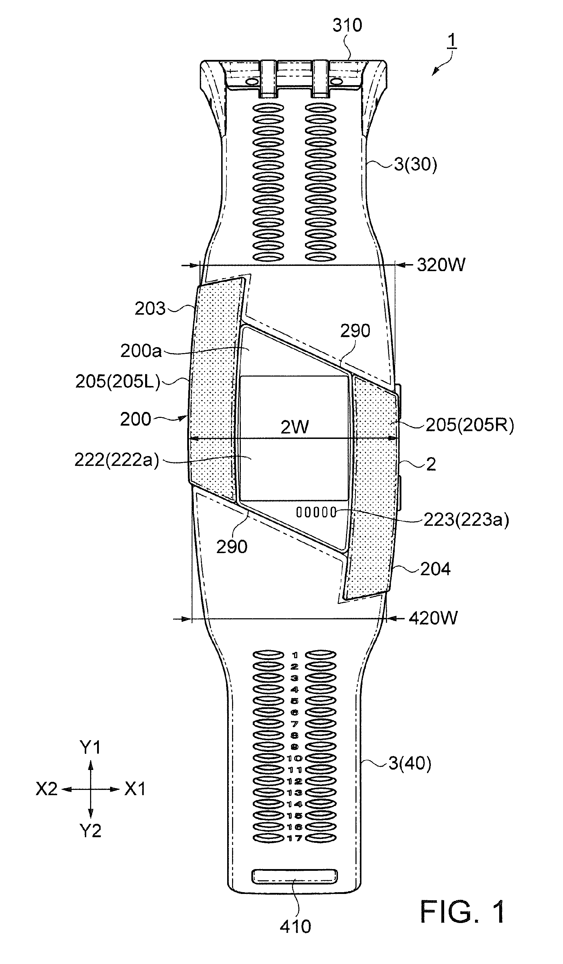

A biological information measurement device according to this embodiment will be described using Figs. 1 to 7C.

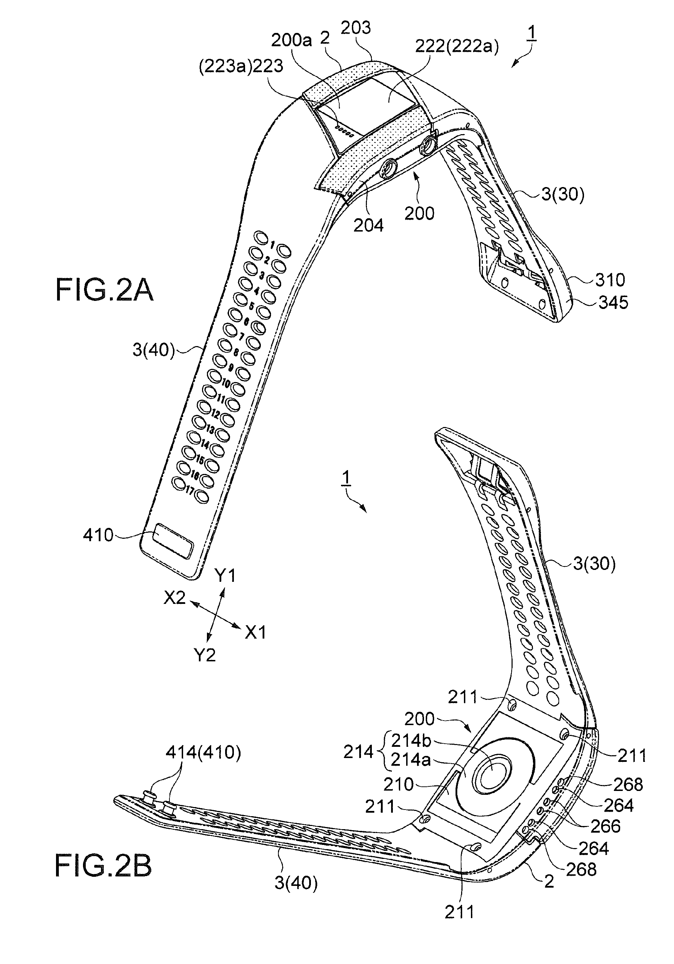

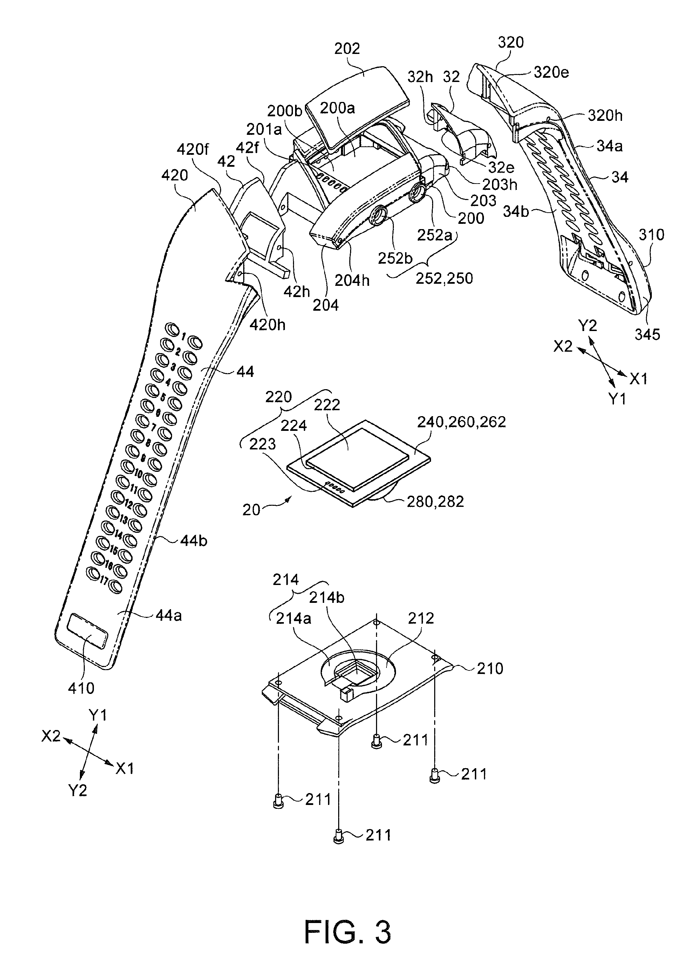

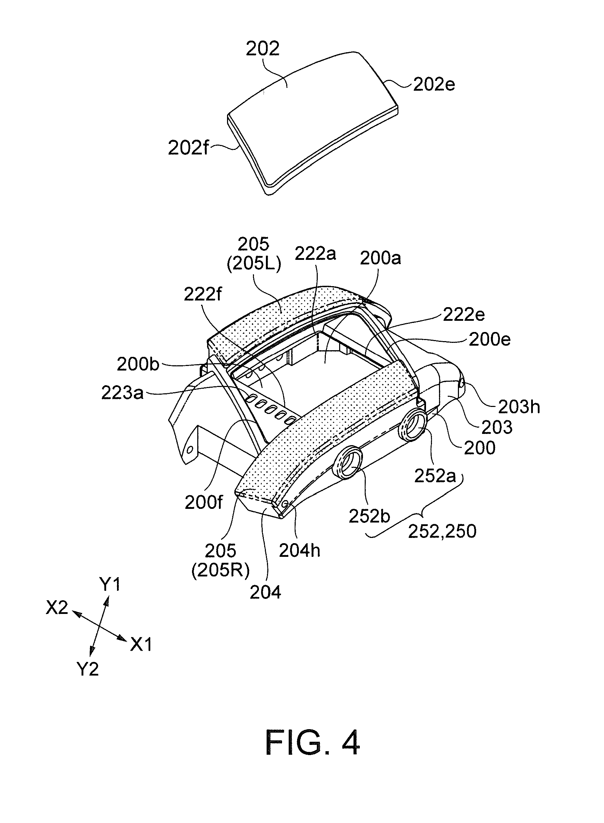

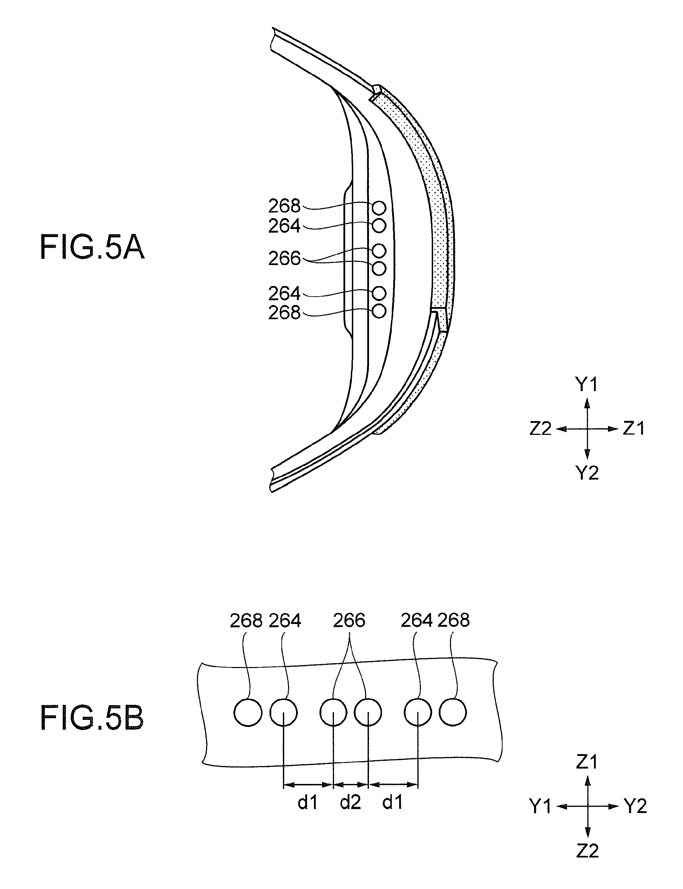

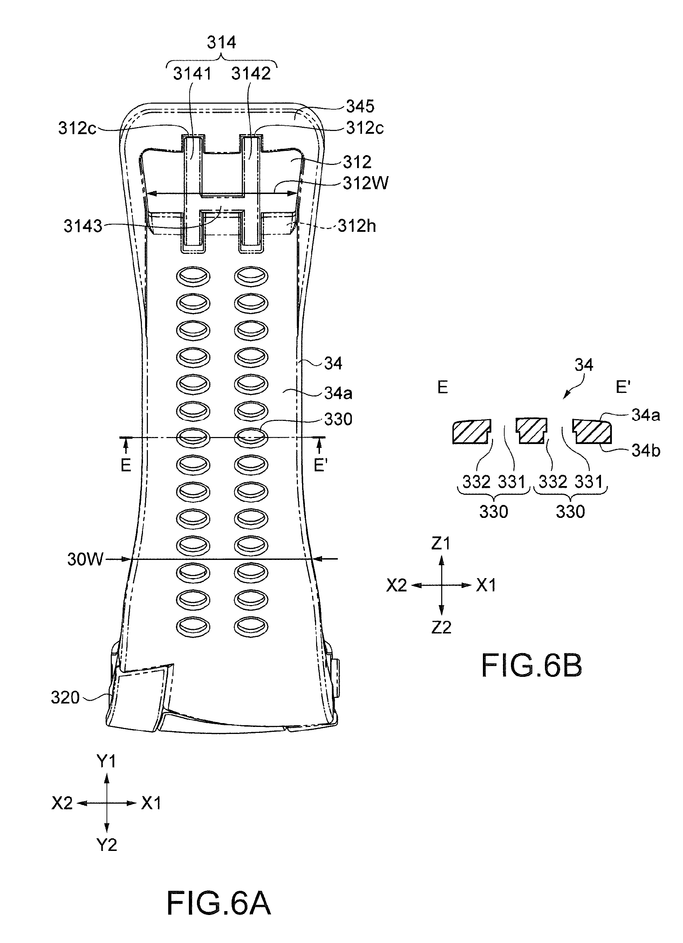

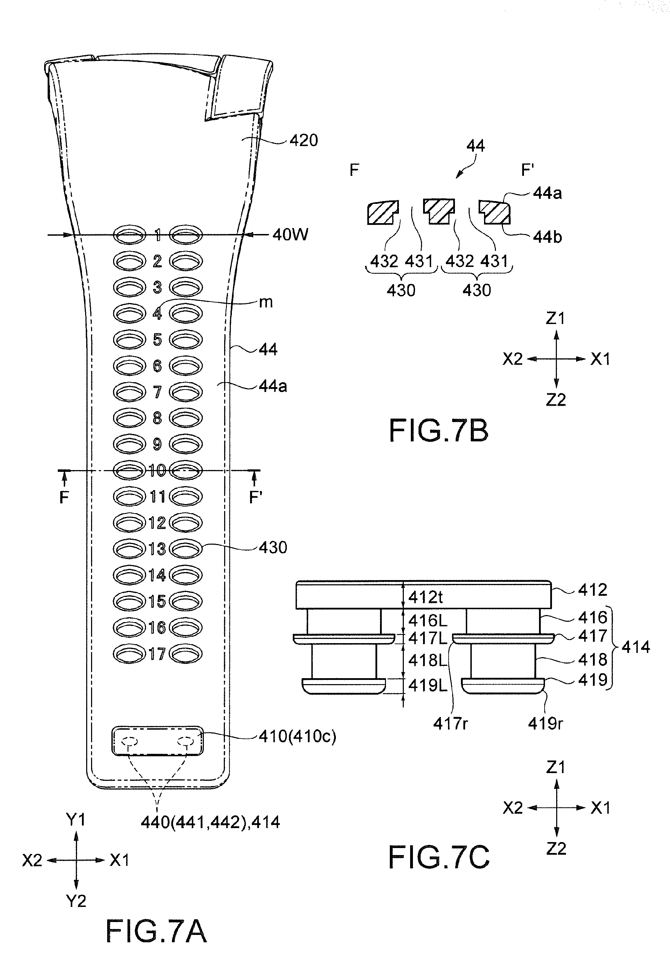

Fig. 1 is a top view schematically showing the external appearance of the biological information measurement device according to this embodiment. Figs. 2A and 2B are perspective views schematically showing the external appearance of the biological information measurement device. Fig. 3 is a development view schematically showing the structure of the biological information measurement device. Fig. 4 is an enlarged view schematically showing the structure of a main body section of the biological information measurement device. Figs. 5A and 5B are a side view schematically showing the nine o'clock-side side surface of the main body section of the biological information measurement device, and an enlarged view of a terminal section. Figs. 6A and 6B are a plan view and a cross-sectional view schematically showing a first band portion which is connected to a device main body. Figs. 7A to 7C are a plan view and a cross-sectional view schematically showing a second band portion which is connected to the device main body.

### Schematic Configuration of Biological Information Measurement Device 1

A biological information measurement device 1 (hereinafter referred to simply as a "measurement device 1") according to this embodiment is an electronic device which is mounted on the wrist or the like of a test object (for example, a human body), biological information of which is measured, and measures biological information such as the pulse. The measurement device 1 is formed to have an external appearance similar to a wristwatch, as shown in Figs. 1, 2A, and 2B. The measurement device 1 is provided with a device main body 2 which comes into close contact with a test object, thereby measuring biological information, and a band 3 which is mounted on the device main body 2.

### Configuration of Device Main Body 2

As shown in Figs. 2A, 2B, and 3, the device main body 2 of the measurement device 1 is provided with a module 20, and a case section 200 in which the module 20 is accommodated. The module 20 is provided with a display section 220, a processing section 240, a power supply section 260, and a sensor section 280.

In addition, the display section 220 is provided with a first display portion 222 and a second display portion 223. Further, the device main body 2 is provided with an operation section 250 connected to the processing section 240, and a communication terminal 266. In addition, the device main body 2 is provided with a charging terminal 264 connected to the power supply section 260.

In the case section 200, a concave portion 200b in which the module 20 is accommodated is formed on the lid section 210 side thereof. The module 20 is accommodated in the concave portion 200b and the lid section 210 is disposed so as to cover the concave portion 200b and is fixed by setscrews 211. Materials of the case section 200 and the lid section 210 are not particularly limited. In this embodiment, as an example thereof, nylon-based synthetic resin (plastic resin) in which the occurrence of cracks are hard is used.

As shown in Figs. 2A, 2B, and 3, in the case section 200, a display window 200a is provided on the opposite side to the side on which the lid section 210 is provided. The display window 200a is configured such that pulse measurement data or the like which is displayed on the display section 220 is visible.

The display window 200a is provided with a first display window 222a corresponding to a first display portion 222, and a second display window 223a corresponding to a second display portion 223. The first display portion 222 is fitted into the first display window 222a (refer to Figs. 1, 2A, and 2B). Further, the second display portion 223 is fitted into the second display window 223a (refer to Figs. 1, 2A, and 2B) .

As shown in Fig. 4, the display window 200a has a parallelogram shape in which an upper base (an upper side) 200e and a lower base (a lower side) 200f which are oblique sides extending in a direction (a second direction, an X direction shown in Figs. 1 to 3) intersecting a direction (a first direction, a Y direction shown in Figs. 1 to 3) in which the band 3 extends from the device main body 2 form a set of opposite sides.

The first display window 222a in which the first display portion 222 is fitted has a quadrangular shape having an upper base (an upper side) 222e and a lower base (a lower side) 222f which extend in the second direction intersecting the first direction in which the band 3 extends from the device main body 2.

The second display window 223a in which the second display portion 223 is fitted has a corner-rounded rectangular shape having parallel lines extending in the first direction in which the band 3 extends from the device main body 2.

Here, the display window 200a is disposed such that the upper base 200e of the display window 200a and the upper base 222e of the first display window 222a come into contact with each other at an acute angle. Further, the display window 200a is disposed such that the lower base 200f of the display window 200a and the lower base 222f of the first display window 222a come into contact with each other at an acute angle.

The second display window 223a is disposed at an area having an internal angle at which the lower base 200f of the display window 200a and the lower base 222f of the first display window 222a come into contact with each other at an acute angle.

The second display portions 223 and the second display windows 223a are provided at the area having the internal angle, whereby it is possible to display an operating state or the like of the measurement device 1 in a plurality of ways. Further, it is possible to make the first display portion 222 (the first display window 222a) be viewed as large when viewing the device main body 2, while suppressing the surface area of the device main body 2. Therefore, it is possible to suppress a feeling of pressure which is given to a person with the measurement device 1 mounted thereon.

Further, a display section cover body 202 formed of transparent resin, transparent glass, or the like is fitted into the display window 200a. The display section 220 is protected by the display section cover body 202. The display section cover body 202 is formed into a parallelogram shape based on the display window 200a. The display section cover body 202 has a parallelogram shape in which an upper base 202e and a lower base 202f which are oblique sides disposed in the first direction form a set of opposite sides.

As shown in Figs. 1 to 4, at the case section 200, a frame section 205 is provided along the display window 200a.

In the frame section 205, a frame portion 205R is provided in an X1 direction (the three o'clock-side direction when a direction in which a first band portion 30 extends is set to be the twelve o'clock direction in a timepiece) shown in Figs. 1 to 4 in the second direction intersecting the first direction in which the band 3 extends from the device main body 2, and a frame portion 205L is provided in an X2 direction (the nine o' clock-side direction when a direction in which a second band portion 40 extends is set to be the six o'clock direction in a timepiece).

The frame section 205 is disposed with the lower base thereof being in contact with the display window 200a (the display section cover body 202) and has an isosceles trapezoid shape in which a side becoming a leg is provided to extend in the second direction. Further, a side becoming the lower base of the frame section 205 is provided to be longer than a set of opposite sides extending in the first direction, which is different from a set of opposite sides in which the upper base 200e and the lower base 200f of the display window 200a make a pair.

In the frame portion 205L, a side becoming a leg disposed in a Y2 direction shown in Figs. 1 to 4 is provided on an extension line of the lower base 200f of the display window 200a. Therefore, in the frame portion 205L, a side becoming a leg provided in a Y1 direction shown in Figs. 1 to 4 is provided further to the Y1 side than an extension line of the upper base 200e of the display window 200a.

In the frame portion 205R, a side becoming a leg provided in the Y1 direction shown in Figs. 1 to 4 is provided on an extension line of the upper base 200e of the display window 200a. Therefore, in the frame portion 205R, a side becoming a leg provided in the Y2 direction is provided further to the Y2 side than an extension line of the lower base 200f of the display window 200a.

At the frame section 205, a film (not shown)which includes metal is provided on the front side of the case section 200 in which the display window 200a is provided. The film is electrically connected to the processing section 240 (described later). The film can be used as an antenna when performing wireless communication with an information processing device (not shown) provided outside the measurement device 1. Further, the film can be used as a so-called touch sensor as the operation section 250 (described later). In addition, the film is provided, whereby it is possible to increase the strength of the case section 200 and thin the wall thickness of the case section 200. Therefore, it is possible to attain a reduction in the weight of the case section 200.

A material configuring the film is not particularly limited as long as it is a material capable of exhibiting performance as an antenna or a material capable of detecting a change in capacitance. In the measurement device 1 according to this embodiment, a material which includes nickel (Ni) is used. Nickel is used in the film, whereby it is possible to secure performance as an antenna, the strength of the case section 200, and performance as a touch switch and increase the design properties of the measurement device 1 by a glossy color which nickel has. In addition, in the measurement device 1 according to this embodiment, in order to increase performance as an antenna, the strength of the case section 200, and the design properties, the film is also provided on the side surface of the frame section 205.

As shown in Fig. 3, the display section 220 is provided in the module 20 accommodated in the case section 200. The first display portion 222 and the second display portion 223 are provided in the display section 220.

In the first display portion 222, biological information such as the number of pulses, time information such as the current time, or the like is displayed according to each display mode. Further, at the first display portion 222, a backlight 224 is provided and can illuminate the first display portion 222.

In the first display portion 222, as long as it is possible to display biological information (mainly, a numeral or a graph configured by a dot matrix) such as the pulse, a display method is not limited. In the measurement device 1, as an example thereof, a liquid crystal display device is used. Further, as for the backlight 224, as long as it is possible to perform illumination to the extent that biological information which is displayed on the first display portion 222 is visible, a luminescence method or a luminescent color is not limited. In the measurement device 1, the illumination of the first display portion 222 is performed by using an electro-luminescence (EL) panel which emits green light.

In the second display portion 223, each operation mode or the like of the measurement device 1 is displayed by the luminescent color or the blinking of the second display portion 223. In the second display portion 223, as long as it is possible to display each operation mode by the blinking or the luminescent color thereof, a display method is not limited. In the measurement device 1, as an example thereof, a light-emitting diode is used.

As shown in Fig. 3, the processing section 240 is provided in the module 20 accommodated in the case section 200.

The processing section 240 is a substrate configured by a semiconductor device such as a microcomputer, or a storage device, and an electronic circuit or the like which realizes a communication function. The display section 220, the operation section 250, the power supply section 260, the sensor section 280, and the film provided at the frame section 205 are connected to the processing section 240. The processing section 240 processes the driving of the sensor section 280 or a signal based on the pulse detected in the sensor section 280, thereby performing the display processing of biological information or the like in the display section 220.

Further, the processing section 240 performs the storing of biological information and performs communication with the information processing device provided outside the measurement device 1, thereby being able to output the stored data.

As shown in Fig. 3, the operation section 250 providing a command to the processing section 240 is provided in the module 20 accommodated in the case section 200.

In the operation section 250, an operation button 252 capable of being pressed is provided. By pressing the operation button 252, it is possible to perform the switching of a pulse measurement mode of displaying, for example, pulse measurement data, a clock mode of displaying the current time or the like, a remaining battery level display mode, a lighting mode of the backlight 224 of the display section 220, or the like.

The operation section 250 is provided in the side surface of the case section 200 in the X1 direction (the three o'clock-side direction) shown in Figs. 1 to 4 in the second direction intersecting the first direction in which the band 3 extends from the device main body 2. The operation section 250 can be provided with a plurality of operation buttons 252. In the operation section 250, it is preferable that at least one operation button 252 is provided on the extension line of the upper base 200e of the display window 200a. In this way, a test object visually recognizes the upper base 200e of the display window 200a, whereby it is possible to easily grasp the position where the operation button 252 is provided, and thus it is possible to prevent an erroneous operation.

Further, by providing the plurality of operation buttons 252 and pressing the operation buttons 252 at substantially the same time, it is possible to perform switching to a setting mode of performing the setting of the measurement device 1. In the measurement device 1 according to this embodiment, as an example thereof, two operation buttons 252a and 252b are provided in the measurement device 1. An operation of the operation section 250 by a test object can be performed by pressing the operation button 252a by an index finger and adding a force associated with the pressing to the case section 200 through a thumb.

Further, an operation can be performed by pressing the operation button 252b by a ring finger and adding a force associated with the pressing to the case section 200 through a thumb. Therefore, it is preferable that an interval at which the operation buttons 252a and 252b are provided is an interval in which fingers (for example, a thumb and an index finger, or a thumb and a ring finger) performing the operation do not overlap, in a range that the fingers performing the operation reach.

As shown in Figs. 5A and 5B, the charging terminal 264 which is used in the charging of the battery 262 installed in the power supply section 260 which serves as the power supply of the measurement device 1 is provided in the case section 200. In addition, the communication terminal 266 which is used in data communication of biological information (data) measured and stored in the measurement device 1, measurement (measuring) setting data, and the like is provided in the case section 200.

The charging terminal 264 and the communication terminal 266 are provided in the side surface of the case section 200 in the X2 direction (the nine o'clock-side direction) shown in Figs. 1, 2A, and 2B in the second direction intersecting the first direction in which the band 3 extends from the device main body 2. Each of the charging terminal 264 and the communication terminal 266 is provided one or in a plurality in order to fulfill a function thereof. In the measurement device 1 according to this embodiment, as an example thereof, two charging terminals 264 and two communication terminals 266 are provided.

In the charging terminal 264 and the communication terminal 266, the communication terminals 266 are provided between the charging terminals 264. In other words, the charging terminal 264 is disposed outside the communication terminal 266 (in the Y direction shown in Fig. 5B). In addition, a positioning hole 268 for a connector (not shown) to be connected to the charging terminal 264 and the communication terminal 266 is provided outside each charging terminal 264 (in the Y direction which is the opposite direction to a direction in which the communication terminal 266 is provided) . In addition, the charging terminal 264 and the communication terminal 266 are provided so as to protrude from the side surface of the case section 200. Further, the positioning hole 268 is provided as a bottomed hole in the side surface of the case section 200.

As shown in Fig. 5B, a distance d1 between the charging terminal 264 and the communication terminal 266 is wider than a distance d2 between the communication terminals 266. In this way, it is possible to suppress short circuits between the charging terminals 264 and between the charging terminal 264 and the communication terminal 266.

As for the charging terminal 264 and the communication terminal 266, it is possible to use a material having electric conductivity. In the measurement device 1 according to this embodiment, as an example thereof, stainless steel is used as materials of the charging terminal 264 and the communication terminal 266. In this way, the charging terminal 264 and the communication terminal 266 can have corrosion resistance. Further, the charging terminal 264 and the communication terminal 266 are provided to protrude from the case section 200 (have a convex lens-shaped convex curved surface), and therefore, it is possible to easily perform cleaning, and thus it is possible to suppress corrosion of the charging terminal 264 and the communication terminal 266 and maintain electric conductivity. Further, the charging terminal 264 and the communication terminal 266 are provided in the side surface different from the side surface in which the operation section 250 is provided, and therefore, it is possible to suppress the fingers operating the operation buttons 252a and 252b from touching the terminals, thereby preventing contamination due to sebum or the like. In addition, in the charging terminal 264 and the communication terminal 266, by plating the surfaces with a conductive material having corrosion resistance, for example, gold or the like, it is possible to further improve the corrosion resistance.

Returning to Fig. 3, the sensor section 280 will be described.

As shown in Fig. 3, the sensor section 280 provided with a sensor unit 282 is provided in the module 20 accommodated in the case section 200. The sensor unit 282 is a light sensor and is provided with a sensor case, and a sensor substrate with a light emitting element and a light receiving element mounted thereon. The sensor unit 282 irradiates light toward the wrist of a test object from the light emitting element such as a light emitting diode (LED) and receives the light reflected by a blood vessel of the wrist by the light receiving element such as a photodiode.

A sensor bank portion 212 in which the sensor section 280 is accommodated is provided at the lid section 210. At least a portion of the sensor section 280 is provided on the sensor bank portion 212 having a disk shape raised from the lid section 210 in the opposite direction to a display direction of the display section 220. The sensor bank portion 212 is provided with a sensor convex portion 214 (refer to Fig. 2B).

The sensor convex portion 214 is provided so as to be pressed against (be brought into contact with) the wrist or the like of a test object with the measurement device 1 mounted thereon. The sensor convex portion 214 is provided with a base portion 214a extending from the lid section 210, and a tip portion 214b which is pressed against a test object.

From this, it is preferable that the base portion 214a of the sensor convex portion 214 is configured with a material having a light shielding property. Further, it is preferable that the tip portion 214b is configured with a material capable of transmitting light which is emitted from the light emitting element. Further, it is preferable that the tip portion 214b has a shape which suppresses irregular reflection of the light and in which a test object does not feel pain at the time of mounting.

Therefore, the base portion 214a is provided by using the same synthetic resin as the lid section 210 and applying light-shielding coloring thereto. Further, the tip portion 214b is provided to have an arc shape by using transparent glass or transparent acrylic resin.

### Configuration of Band 3

The configuration of the band 3 will be described using Figs. 1 to 3, 6A, and 6B.

The band 3 is provided in order to mount the device main body 2 on a test object. The bands 3 are provided at both ends of the device main body 2, as shown in Figs. 1, 2A, and 2B. The first band portion 30 configuring the band 3 is mounted on a lug 203 (a lug on the twelve o'clock side in a timepiece as the first direction) of the device main body 2 by a mounting member 32, as shown in Fig. 3. Further, the second band portion 40 configuring the band 3 is mounted on a lug 204 (a lug on the six o'clock side in a timepiece as the first direction) of the device main body 2 by a mounting member 42.

A connection portion 310 which connects the first band portion 30 and the second band portion 40 is provided at the first band portion 30. The connection portion 310 is provided at an end portion of the first band portion 30 on the opposite side to the device main body 2 side. Further, a hook portion 410 which locks the second band portion 40 to the first band portion 30 is provided at the second band portion 40. The hook portion 410 is provided at an end portion of the second band portion 40 on the opposite side to the device main body 2 side.

In the following description, in the first band portion 30, the device main body 2 side is described as being referred to as "one end side" and the side on which the connection portion 310 is provided on the opposite side thereto is described as being referred to as the "other end side". Similarly, in the second band portion 40, the device main body 2 side is described as being referred to as "one end side" and the side on which the hook portion 410 is provided on the opposite side thereto is described as being referred to as the "other end side".

### Configuration of First Band Portion

The first band portion 30 shown in Figs. 3, 6A, and 6B has a belt portion 34, a cover portion 320 as a first connection portion (a connection portion) provided on one end side (the device main body 2 side, in the Y2 direction shown in Figs. 3 and 6A) of the belt portion 34, and the connection portion 310 on the other end side (the opposite side to the device main body 2, in the Y1 direction shown in Figs. 3 and 6A).

The belt portion 34 has front and back surfaces. In the following description, the surface coming into contact with a wrist when having been mounted on a test object is described as being referred to as a back surface 34b of the belt portion 34, and the surface being the opposite surface thereto and being visible when having been mounted is described as being referred to as a front surface 34a of the belt portion 34.

### Mounting on Device Main Body 2

The first band portion 30 is mounted on the device main body 2 such that the cover portion 320 covers the lug 203, with the mounting member 32 sandwiched between the lug 203 and the cover portion 320.

The cover portion 320 and the lug 203 are pivotally supported by inserting a "spring rod" (not shown) into a hole 32h provided in the mounting member 32 and a "rod hole 203h" provided in the lug 203 and locking both ends of the spring rod to locking holes 320h provided in the cover portion 320.

In the first band portion 30, if the belt portion 34 having stretchability (described later) and the device main body 2 are directly connected, there is a concern that the connection strength thereof may be lowered. Therefore, the first band portion 30 is mounted on (connected to) the device main body 2 through the cover portion 320, whereby the connection can be made solidly compared to a case of directly connecting the belt portion 34 and the device main body 2.

Here, the connection between the device main body 2 and the first band portion 30 is performed with a miter joint (refer to Fig. 1) making the upper base 202e of the display section cover body 202 (the upper base 200e of the display window 200a) which is the oblique side of the device main body 2 and end portions 320e and 32e which are oblique sides of the cover portion 320 as the connection portion and the mounting member 32 face each other.

The mounting member 32 and the cover portion 320 are formed along the upper base 200e of the display window 200a provided in the case section 200. Further, in the measurement device 1, a bottomed groove 290 is provided between the first band portion 30 (the end portions 320e and 32e) and the device main body 2 (the upper base 200e). The groove 290 is provided, whereby it is possible to suppress wear due to the contact of the case section 200 with the mounting member 32 and the cover portion 320 when the belt portion 34 (described later) expands or contracts. Further, it is possible to suppress detachment of the first band portion 30 due to the contact of the case section 200 with the mounting member 32 and the cover portion 320.

### Belt Portion 34

The belt portion 34 shown in Figs. 6A and 6B has stretchability in order to mount the device main body 2 in close contact with the wrist or the like of a test object. The belt portion 34 is made using a material which includes polyurethane resin or silicone resin, thereby having stretchability and flexibility according to the characteristics of the material.

The belt portion 34 is provided with the thickness (in a Z direction shown in Fig. 6B) of a central portion in a direction of a width 30W increased in a cross-section (a cross-section in line E - E' shown in Fig. 6A) in a direction (the second direction, the X direction shown in Fig. 6A) intersecting a direction (the first direction, the Y1 direction shown in Fig. 6A) in which the first band portion 30 extends from the device main body 2. The belt portion 34 is provided with the thickness of the central portion in the direction of the width 30W, that is, a Z1 direction increased, whereby strength at the time of expansion and contraction and strength at the time of flexion are secured.

A hole portion 330 in which the hook portion 410 provided at the second band portion 40 is locked is provided in the belt portion 34. The hole portions 330 are provided in parallel in the direction of the width 30W (the second direction, the X direction shown in Figs. 6A and 6B) and to be aligned to form rows in a direction (the first direction, the Y direction shown in Fig. 6A) in which the belt portion 34 extends. A line spacing of the hole portions 330 may be disposed so as to be almost constant and may also be disposed such that the line spacing becomes shorter as it goes in a direction of the device main body 2, that is, the Y2 direction from the other end. In other words, the line spacing is disposed such that a distance (a second pitch) between the last row in the Y2 direction and a row adjacent thereto becomes narrow compared to a distance (a first pitch) between the last row of the hole portion 330 on the Y1 direction side and a row adjacent thereto. Due to such a configuration, even for a person in which a site of mounting the measurement device 1 is thin, it is possible to finely adjust tightening, and therefore, it becomes easy to perform setting so as to be able to set the measurement device with an appropriate strength.

In the hole portion 330, as shown in Fig. 6B, a hole 331 provided on the front surface 34a side of the belt portion and a hole 332 provided on the back surface 34b side of the belt portion are coaxially provided.

The hole portion 330 has an elliptical shape having a long axis (a major axis) in the direction of the width 30W (the X direction) of the belt portion 34 and a short axis (a minor axis) in an orthogonal direction (the Y direction) in which the first band portion 30 extends from the device main body 2. The long axis and the short axis orthogonal to the long axis of the hole Portion 330 are provided to be longer (larger) in the hole 332 than in the hole 331.

### Connection Portion 310

The connection portion 310 is provided on the other end side of the belt portion 34 (in the Y1 direction shown in Fig. 6A).

A thick portion 345 in which the thickness of the belt portion 34 is increased compared to a portion in which the hole portions 330 are provided is provided on the other end side of the belt portion 34. The connection portion 310 is provided at the thick portion 345 in which the thickness of the belt portion 34 is increased. An insertion hole 312 into which the second band portion 40 is inserted and a projecting bar 314 which is inserted into a hole portion 430 (refer to Figs. 7A to 7C) provided in the second band portion 40 are provided at the connection portion 310.

In the connection portion 310, the band insertion hole 312 is provided such that a width 312W in a direction (the X direction shown in Fig. 6A) intersecting a direction (the Y1 direction shown in Fig. 6A) in which the first band portion 30 extends from the device main body 2 is wider than a width 40W (refer to Fig. 7A) of the second band portion 40.

More specifically, a width on a side of one side (the Y1 side shown in Fig. 6A) on which a concave portion 312c (described later) is provided is provided to be wider than a width on the side of one side (the Y2 side shown in Fig. 6A) on which a locking hole 312h in which the projecting bar 314 (described later) is locked is provided.

The band insertion hole 312 is provided such that the width on the Y1 side is wider, whereby it is possible to easily insert the second band portion 40 therein. Further, it is possible to increase the close contact of the device main body 2 with a wrist by extending the second band portion 40 by pulling the second band portion 40 to the Y2 side with it being brought into contact with an edge of the band insertion hole 312 on the Y1 side on which the width is provided to be wide, and adjust tightening by the restoring force of the second band portion 40 (a belt portion 44).

The projecting bar 314 is pivotally supported on the belt portion 34 by a "spring rod" (not shown) which is inserted into the locking hole 312h provided at one side on the Y2 side at an inner edge of the band insertion hole 312, and a hole (not shown) provided in the projecting bar 314. A portion of the projecting bar 314 is fitted into the concave portion 312c provided at one side on the Y1 side at the inner edge of the band insertion hole 312, whereby the projecting bar 314 is locked to the belt portion 34.

In the projecting bar 314, two bar portions 3141 and 3142 are provided to extend in the Y1 direction from the side of one side on the Y2 side at the inner edge of the band insertion hole 312. Further, in the projecting bar 314, a connection bar 3143 is provided in the direction of the width 30W (the X direction shown in Fig. 6A) of the belt portion 34 intersecting the Y1 direction in which the bar portions 3141 and 3142 extend. The cross-sectional shape of each of the bar portions 3141 and 3142 configuring the projecting bar 314 has a flat shape. The projecting bar 314 has a flat shape, whereby it is possible to increase a contact area when having been inserted into the hole portion 430, suppress the hole portion 430 from being extended, and suppress a shift of the measurement device 1 mounted on a test object by the band 3 according to this embodiment.

A material configuring the projecting bar 314 is not particularly limited, and it is acceptable if it is a material capable of withstanding the restoring forces of the belt portions 34 and 44. In this embodiment, as for the projecting bar 314, as an example thereof, stainless steel is used. The projecting bar 314 can have toughness to withstand the restoring forces of the belt portions 34 and 44 and corrosion resistance by using stainless steel. Further, the projecting bar 314 is subjected to hairline machining along a direction (the Y direction) in which the bar portions 3141 and 3142 extend. By performing the hairline machining, it is possible to increase the visibility of the projecting bar 314, thereby aiding insertion into the hole portion 430.

In the band 3 according to this embodiment, the second band portion 40 is inserted into the band insertion hole 312 and the projecting bar 314 is inserted into the hole portion 430 provided in the second band portion 40, whereby the first band portion 30 and the second band portion 40 are connected.

Here, it is required that the band 3 brings the device main body 2 into close contact with a test object in order to measure the biological information of the test object and the mounting position thereof is not shifted.

For this reason, the hole portions 430 are provided in parallel in the second band portion 40, and the projecting bar 314 is inserted into the hole portion 430, whereby a shift of a mounting position is suppressed (refer to Figs. 7A to 7C) . In the connection portion 310, the bar portions 3141 and 3142 configuring the projecting bar 314 are provided in parallel corresponding to the hole portions 430 and an interval thereof is maintained by the connection bar 3143. In this way, the projecting bar 314 has an H-shape, and thus it is possible to easily insert the projecting bar 314 into the hole portions 430 provided in parallel, while maintaining an interval between the bar portions 3141 and 3142 by the connection bar 3143.

### Cover Portion 320

The cover portion 320 as the first connection portion is provided on one end side (in the Y2 direction shown in Fig. 6A) of the belt portion 34. The locking hole 320h is provided in the cover portion 320.

The cover portion 320 is provided in order to connect the first band portion 30 to the device main body 2 through the mounting member 32. A width 320W of the cover portion 320 and a width 2W of the device main body 2 have approximately the same width (refer to Fig. 1).

More specifically, in a direction (the X direction shown in Figs. 1 to 3, or Figs. 6A and 6B) intersecting a direction (the Y1 direction shown in Figs. 1 to 6A) in which the first band portion 30 extends from the device main body 2, the width 320W of the cover portion 320 and the width 2W of the device main body 2 have approximately the same width. Further, the cover portion 320 is formed into a shape having the end portion 320e which becomes an oblique side along the upper base 200e of the display window 200a formed into a parallelogram shape (refer to Fig. 3). In this way, the device main body 2 and the first band portion 30 can be visible as being visually integrally formed, and thus it is possible to alleviate a feeling of pressure due to mounting. Further, since the device main body 2 does not protrude from the first band portion 30 in the width direction (the X direction), it is possible to suppress the clothes of a test object from being caught in the device main body 2 at the time of mounting.

### Configuration of Second Band Portion

The second band portion 40 shown in Figs. 3, and 7A to 7C has the belt portion 44, the cover portion 420 as a second connection portion (a connection portion) provided on one end side (the device main body 2 side, in the Y1 direction shown in Figs. 3 and 7A) of the belt portion 44, and the hook portion 410 on the other end side (the opposite side to the device main body 2, in the Y2 direction shown in Figs. 3 and 7A). In addition, in Fig. 7A, illustration of a concave portion 410c in which the hook portion 410 is provided and a hole portion 440 is simplified.

The belt portion 44 has front and back surfaces, and in the following description, the surface coming into contact with a wrist when having been mounted on a test object is described as being referred to as a back surface 44b of the belt portion 44 and the surface being the opposite surface thereof and being visible when having been mounted is described as being referred to as a front surface 44a of the belt portion 44.

### Mounting on Device Main Body 2

The second band portion 40 is mounted on the device main body 2 such that the cover portion 420 covers the lug 204, with the mounting member 42 sandwiched between the lug 204 and the cover portion 420.

The cover portion 420 and the lug 204 are pivotally supported by inserting a "spring rod" (not shown) into a hole 42h provided in the mounting member 42 and a "rod hole 204h" provided in the lug 204 and locking both ends of the spring rod to locking holes 420h provided in the cover portion 420.

In the second band portion 40, if the belt portion 44 having stretchability (described later) and the device main body 2 are directly connected, there is a concern that the connection strength thereof may be lowered. Therefore, the second band portion 40 is mounted on (connected to) the device main body 2 through the cover portion 420, whereby the connection can be made solidly compared to a case of directly connecting the belt portion 44 and the device main body 2.

Here, the connection between the device main body 2 and the second band portion 40 is performed with a miter joint (refer to Fig. 1) making the lower base 202f of the display section cover body 202 (the lower base 200f of the display window 200a) which is the oblique side of the device main body 2 and end portions 420f and 42f which are oblique sides of the cover portion 420 as the connection portion and the mounting member 42 face each other.

The mounting member 42 and the cover portion 420 are formed along the lower base 200f of the display window 200a provided in the case section 200. Further, in the measurement device 1, the bottomed groove 290 is provided between the second band portion 40 (the end portions 420f and 42f) and the device main body 2 (the lower base 200f). The groove 290 is provided, whereby it is possible to suppress wear due to the contact of the case section 200 with the mounting member 42 and the cover portion 420 when the belt portion 44 (described later) expands or contracts. Further, it is possible to suppress detachment of the second band portion 40 due to the contact of the case section 200 with the mounting member 42 and the cover portion 420.

### Belt Portion 44

The belt portion 44 shown in Figs. 7A and 7B has stretchability in order to mount the device main body 2 in close contact with the wrist or the like of a test object. The belt portion 44 is made using a material which includes polyurethane resin or silicone resin, thereby having stretchability and flexibility according to the characteristics of the material.

The belt portion 44 is provided with the thickness (in the Z direction shown in Fig. 7B) of a central portion in a direction of the width 40W increased in a cross-section (a cross-section in line F - F' shown in Fig. 7A) in a direction (the second direction, the X direction shown in Fig. 7A) intersecting a direction (the first direction, the Y2 direction shown in Fig. 7A) in which the second band portion 40 extends from the device main body 2. The belt portion 44 is provided with the thickness of the central portion in the direction of the width 40W increased, whereby strength at the time of expansion and contraction and strength at the time of flexion are secured.

The hole portion 430 in which the projecting bar 314 provided at the first band portion 30 is inserted is provided in the belt portion 44. The hole portions 430 are provided in parallel in the direction of the width 40W (the second direction, the X direction shown in Figs. 7A to 7C) and to be aligned to rows in a direction (the first direction, the Y direction shown in Figs. 7A and 7B) in which the belt portion 44 extends. A line spacing of the hole portions 430 may be disposed so as to be almost constant and may also be disposed such that the line spacing becomes shorter as it goes in a direction from the other end to the device main body 2, that is, the Y1 direction. In other words, the line spacing is disposed such that a distance (a second pitch) between the last row in the Y1 direction and a row adjacent thereto becomes narrow compared to a distance (a first pitch) between the last row of the hole portion 430 on the Y2 direction side and a row adjacent thereto. Due to such a configuration, even with respect to a person in which a site of mounting the measurement device 1 is thin, it is possible to finely adjust tightening, and therefore, it becomes easy to set the measurement device 1 with an appropriate strength.

In the hole portion 430, as shown in Fig. 7B, a hole 431 provided on the front surface 44a side of the belt portion 44 and a hole 432 provided on the back surface 44b side of the belt portion 44 are coaxially provided.

The hole portion 430 has an elliptical shape having a long axis (a major diameter) in the direction of the width 40W (the X direction) of the belt portion 44 and a short axis (a minor diameter) in an orthogonal direction (the Y direction) in which the second band portion 40 extends from the device main body 2, as shown in Fig. 7B. The long axis and the orthogonal short axis of the hole portion 430 are provided to be longer (larger) in the hole 432 than in the hole 431.

In addition, the elliptical shape that the holes 431 and 432 have is a shape according to the cross-sectional shape of the projecting bar 314 provided at the connection portion 310 described above and may be appropriately changed in accordance with the cross-sectional shape of the projecting bar 314. The holes 431 and 432 have an elliptical shape, thereby being able to be easily deformed, and thus the insertion of the projecting bar 314 can be easily performed. In addition, the holes 431 and 432 also have an excellent restoring force after the deformation.

In the belt portion 44, as shown in Fig. 7A, symbol m is denoted between the hole portions 430 provided in parallel. The symbols m are denoted to form order toward the direction (the Y2 direction shown in Fig. 7A) in which the second band portion 40 extends from the device main body 2. The symbols m are denoted as "1, 2, 3, ..., and 17" in order from the device main body 2 side (the Y1 side shown in Fig. 7A) in a case of being, for example, a numeral. Further, in a case of being an alphabet letter, the symbols can be denoted as "a, b, c, ..." in order from the device main body 2 side. In a case of using an alphabet letter, compared to a case of using a numeral, it is possible to indicate a permutation of a lot of hole portions 430 with the same letter size. That is, it is possible to indicate a permutation of the hole portions 430 of the number equivalent to the twenty-five letters of the alphabet with one letter while maintaining the size of the letter. The font of the symbol m is not particularly limited as long as it is possible to indicate the order of the hole portions 430.

The symbols m are denoted on the belt portion 44, whereby, when repeatedly mounting the measurement device 1 on a wrist or the like, it is possible to easily identify the optimum position for inserting the projecting bar 314 in order to connect the first band portion 30 and the second band portion 40, and thus it is possible to mount the measurement device 1 without seeking the position of the hole portion 430 for inserting the projecting bar 314 which is optimum for each mounting. Further, the symbol m is denoted between the hole portions 430 provided in parallel, and therefore, it is possible to denote the symbol to the second band portion 40 without newly providing a space (an area).

Further, as shown in Fig. 7A, a concave portion (not shown) in which a base portion 412 of the hook portion 410 (described later) is fitted and the hole portion 440 in which a pin 414 extending the base portion 412 is inserted are provided in the belt portion 44.

The concave portion has a concave shape conforming to an outer peripheral edge of the base portion 412 on the front surface 44a of the belt portion. Further, a depth of the concave portion is provided to be approximately the same dimension as a thickness 412t of the base portion 412.

The hole portions 440 are provided in parallel in the direction of the width 40W (the X direction) and coaxially with the pins 414 extending from the base portion 412, as shown in Fig. 7A. In the hole portion 440, a hole (not shown) provided in a bottom portion of the concave portion on the front surface 44a side of the belt portion 44 and a hole (not shown) provided in the back surface 44b of the belt portion 44 are coaxially provided.

### Hook Portion 410

As shown in Figs. 1 to 3 or Figs. 7A to 7C, the hook portion 410 is provided on the other end side (in the Y2 direction shown in Figs. 1 to 3 or Fig. 7A) of the belt portion 44.

As shown in Fig. 7C, the hook portion 410 is provided with the base portion 412 and the pins 414 extending from the base portion 412. The pin 414 is configured to include a first shaft portion 416 and a second shaft portion 418. The hook portion 410 is a member for locking the second band portion 40 inserted into the connection portion 310 to the first band portion 30 by inserting the pins 414 into the hole portions 330 provided in the first band portion 30.

In the hook portion 410, the plurality of pins 414 are provided in accordance with an interval of the hole portions 330 provided in parallel in the direction of the width 30W (the X direction shown in Figs. 1 to 3) of the belt portion 34.

Since the pin 414 is inserted into the hole portion 440 provided in the second band portion 40 and the hole portion 330 of an elliptical shape provided in the first band portion 30, the cross-sectional shape thereof is an elliptical shape. The pin 414 is formed into an elliptical shape having a long axis (a major diameter) in the direction of the width 30W of the belt portion 34 (the direction of the width 40W of the belt portion 44) and a short axis (a minor diameter) in an orthogonal direction (the Y direction) in which the second band portion 40 extends from the device main body 2.

In the hook portion 410, the first shaft portion 416 configuring the pin 414 is provided so as to protrude from the base portion 412. Further, the second shaft portion 418 is provided at one end (in a Z2 direction shown in Fig. 7C, one end on the opposite side to the base portion 412 from which the first shaft portion 416 extends) of the first shaft portion 416.

A first head portion 417 is provided at the first shaft portion 416. The first head portion 417 is provided on the opposite side (in the Z2 direction shown in Fig. 7C) to the base portion 412 from which the first shaft portion 416 extends. The first head portion 417 has a curved surface 417r at an outer peripheral edge on the side (in the Z2 direction shown in Fig. 7C) on which the second shaft portion 418 is provided.

The diameter of the first head portion 417 is provided to be larger than the diameter of the first shaft portion 416. Further, the diameter of the first head portion 417 is provided to be larger than the diameter of a hole 441 and smaller than the diameter of a hole 442.

Further, a second head portion 419 is provided at the second shaft portion 418. The second head portion 419 is provided on the opposite side (in the Z2 direction shown in Fig. 7C) to the first shaft portion 416 from which the second shaft portion 418 extends. The second head portion 419 has a curved surface 419r at an outer peripheral edge on the opposite side (in the Z2 direction shown in Fig. 7C) to the side on which the first shaft portion 416 is provided.

The diameter of the second head portion 419 is provided to be larger than the diameter of the second shaft portion 418.

Further, the diameter of the first head portion 417 is provided to be larger than the diameter of the hole 331 provided in the hole portion 330 in which the second head portion 419 is inserted and smaller than the diameter of the hole 332.

Here, the thickness (in a direction in which the pin 414 extends, the thickness in the Z direction shown in Fig. 7C) of the base portion 412 and the length (in the direction in which the pin 414 extends, the length in the Z direction shown in Fig. 7C) of the pin 414 are determined depending on the depths of the hole portion 440 and the hole 331 (the hole portion 330).

The thickness 412t of the base portion 412 in this embodiment is approximately the same as the depth of the concave portion. A length 416L of a shaft portion of the first shaft portion 416 is approximately the same as the depth (the length) of the hole 441. A length (a thickness) 417L of the first head portion 417 of the first shaft portion 416 is approximately the same as the depth (the length) of the hole 442. A length 418L of a shaft portion of the second shaft portion 418 is approximately the same as the depth (the length) of the hole 331. A length (a thickness) 419L of the second head portion 419 of the second shaft portion 418 is approximately the same as the depth (the length) of the hole 332.

In the hook portion 410, the base portion 412 is fitted into the concave portion 410c provided in the front surface 44a of the belt portion 44. In addition, the pins 414 extending from the base portion 412 are inserted into the hole portions 440 provided in parallel in the direction of the width 40W of the belt portion 44, whereby the hook portion 410 is provided at the belt portion 44. The hook portion 410 is provided such that the second shaft portions 418 protrude from the back surface 44b of the belt portion 44 when having been provided at the belt portion 44. The second shaft portions 418 protrude from the belt portion 44, thereby being able to be inserted into the hole portions 330 provided in the first band portion 30 (the belt portion 34).

In this way, in the first shaft portion 416 inserted into the hole 441, the first head portion 417 is caught in the hole 441, and thus it is possible to suppress the hook portion 410 from being detached from the belt portion 44.

More specifically, due to the curved surface 417r provided at the first head portion 417, it is possible to reduce contact resistance occurring when inserting the first shaft portion 416 into the hole 441. In addition, the curved surface 417r is not provided at the first head portion 417 on the base portion 412 side, and therefore, when extracting the first shaft portion 416 from the hole 441, a larger force than the force at the time of insertion is required. Therefore, it is possible to suppress the hook portion 410 from being detached from the belt portion 44.

Further, in the second shaft portion 418 inserted into the hole portion 330, the second head portion 419 is caught in the hole 331 provided in the first band portion 30, and thus it is possible to lock the second band portion 40 to the first band portion 30. Further, it is possible to suppress detachment of the second band portion 40 locked to the first band portion 30.

More specifically, due to the curved surface 419r provided at the second head portion 419, it is possible to reduce contact resistance occurring when inserting the second shaft portion 418 into the hole 331. In addition, the curved surface 419r is not provided at the second head portion 419 on the first shaft portion 416 side, and therefore, when extracting the second shaft portion 418 from the hole 331, a larger force than the force at the time of insertion is required. Therefore, it is possible to suppress detachment of the second band portion 40 locked to the first band portion 30.

### Cover Portion 420

The cover portion 420 as the second connection portion is provided on one end side (in the Y1 direction shown in Fig. 7A) of the belt portion 44. The locking hole 420h is provided in the cover portion 420.

The cover portion 420 is provided in order to connect the second band portion 40 to the device main body 2 through the mounting member 42. A width 420W of the cover portion 420 and the width 2W of the device main body 2 have approximately the same width (refer to Fig. 1).

More specifically, in a direction (the X direction shown in Figs. 1 to 3, or Figs. 7A to 7C) intersecting the direction (the Y1 direction shown in Figs. 1 to 3, or Fig. 7A) in which the second band portion 40 extends from the device main body 2, the width 420W of the cover portion 420 and the width 2W of the device main body 2 have approximately the same width. Further, the cover portion 420 is formed into a shape having the end portion 420f which becomes an oblique side along the lower base 200f of the display window 200a formed into a parallelogram shape (refer to Fig. 3). In this way, the device main body 2 and the second band portion 40 can be visible as being visually integrally formed, and thus it is possible to alleviate a feeling of pressure due to mounting. Further, since the device main body 2 does not protrude from the second band portion 40 in the width direction (the X direction), it is possible to suppress the clothes of a wearer from being caught in the device main body 2 at the time of mounting.

According to the embodiment described above, the following effects can be obtained.

According to the measurement device 1, the device main body 2 and the band 3 are connected by a miter joint. Since the device main body 2 and the band 3 are connected by a miter joint, it is possible to cause the widths of the device main body 2 and the band 3 to continuously change. Therefore, it is possible to make the device main body 2 be viewed as small, and thus it is possible to suppress (alleviate) a visual feeling of pressure due to prolonged wearing. Therefore, it is possible to realize the measurement device 1 in which the influence on the biological information of a test object with the measurement device mounted thereon is suppressed, and thus it is possible to obtain biological information with high precision. Further, the shape of the measurement device 1 changes continuously and smoothly, whereby, even when a test object is in motion with the device main body 2 mounted on an arm or the like, it is possible to suppress the device main body 2 from being caught in a sleeve of clothing. In addition, it is possible to make the device main body 2 be viewed as small, whereby it is possible to suppress (alleviate) a visual feeling of pressure due to prolonged wearing, and thus it is possible to measure biological information over a longer period of time. By continuing to measure the biological information of a test object over a long period of time, it becomes easy for a test object to more accurately grasp one's own state.

In addition, in this embodiment, an example having a function of measuring biological information has been described. However, the invention is not limited thereto, and it is possible to carry out the invention as a portable electronic information device which collects and displays a variety of information. By varying the configuration of the module 20 configuring the device main body 2, it is possible to apply the invention as, for example, a navigation device or a communication device.

## Claims

1. A biological information measurement device comprising:
a device main body; and
a band connected to the device main body,
wherein oblique sides are provided at end portions of the device main body to which the band is connected and the band, and
the band is connected to the device main body with the oblique side of the device main body and the oblique side of the band facing each other.

2. The biological information measurement device according to Claim 1, further comprising: a connection portion which connects the device main body and the band.

3. The biological information measurement device according to Claim 1 or 2, wherein a bottomed groove portion is provided between the oblique side of the device main body and the oblique side of the band.

4. The biological information measurement device according to any one of the preceding claims, wherein the device main body is provided with a case in which a module with an electronic circuit installed therein is accommodated and has a front and a back, and
an area provided with a film which includes metal is provided on a front surface of the case, and the film and the module are electrically connected.

5. The biological information measurement device according to Claim 4, wherein the module is provided with a first display portion fitted into a window portion having a quadrangular shape provided with an upper base and a lower base extending in a second direction intersecting a first direction in which the band connected to the device main body extends, on the front surface of the case, and
the first display portion is covered with a cover body having a parallelogram shape provided with an upper base and a lower base becoming a set of opposite sides extending in the second direction.

6. The biological information measurement device according to Claim 5, wherein the opposite sides of the cover body are disposed side by side with the oblique sides on the connection portion side, and the opposite sides are in contact with the upper base and the lower base of the window portion at an acute angle.

7. The biological information measurement device according to any one of claims 4 to 6, wherein the module is provided with a second display portion fitted into a hole portion provided in the front surface of the case, and the second display portion is covered with the cover body.

8. The biological information measurement device according to Claim 7, wherein the second display portion is provided at an area on an internal angle side in which the lower side of the window portion and the opposite side are in contact with each other at an acute angle.

9. The biological information measurement device according to any one of the preceding claims, wherein the module has a control section, an operation section providing a command is connected to the control section, and
the operation section is provided at least one in a three o'clock-side side surface of the case section in a case where the window portion is viewed in plan from a vertical direction and on an extension line of the opposite side.

10. The biological information measurement device according to any one of the preceding claims, wherein the module is provided with a power supply section having a battery which drives the control section or the first display portion and the second display portion,
a terminal for communication to output data stored in the control section to the outside and a terminal for charging to charge the battery are connected to the module, and
the terminals are disposed in a nine o'clock-side side surface of the case section in a case where the window portion is viewed in plan from a vertical direction.

11. The biological information measurement device according to Claim 10, wherein the terminals are configured to include two or more terminals for charging and two or more terminals for communication, and a distance between the terminals for charging and a distance between the terminals for communication are different from each other.

12. The biological information measurement device according to Claim 10 or 11, wherein in the terminals, the terminal for charging is disposed outside the terminal for communication.

13. The biological information measurement device according to Claim 11 or 12, wherein a material which includes stainless steel is used for the terminals.

14. The biological information measurement device according to any one of claims 10 to 13, wherein tips of the terminals are exposed on a surface of the case or protrude from the surface of the case.

15. The biological information measurement device according to any one of claims 4 to 14, wherein the case section is provided with a positioning hole which is used when the terminal is connected to an external device, and the positioning hole is disposed on the same line as the terminals.
